# EUROPEAN PATENT APPLICATION

(11) **EP 2 277 544 A1**
(43) Date of publication of application: **26.01.2011**
(21) Application number: 09008893.1
(22) Date of filing: 08.07.2009
(51) Int. Cl.: A61K 41/00, A61K 9/50, A61K 47/48, A61K 49/00, A61K 49/18, A61K 31/137

(54) **Biocompatible magnetic nano-clusters containing iron oxide respectively iron oxide - boron with primary use in magnetic drug targeting and boron neutron capture therapy**

(71) Applicant: Ciobanu, Nelica, 80809 München (DE)
(72) Inventor: Ciobanu, Nelica, 80809 München (DE)

(57) **Abstract**

The invention offers a preparation method for biocompatible magnetic nano-clusters containing iron oxide respectively iron oxide - boron. The nano-clusters have a spherical-like shape and consist of close packed small grains of iron oxide respectively iron oxide-boron contained in an iron-based amorphous matrix. The magnetic nano-clusters can be prepared with size distributions around 200 nm, 150 nm, 100 nm, 50 nm, 15 nm and 5 nm. The boron content is tunable in the range 10⁻⁴ wt% (ppm) to 10⁻¹ wt%. The matrix becomes hydrophilic after the attachment of organic molecules. Supplementary molecules like i.e. anticancer drugs can be loaded on the nano-clusters. The magnetic nano-clusters have a large magnetic moment per nano-cluster and a saturation magnetization in the range 35-64 emu/g. The collection in external magnetic fields with maximum of 1-2 T is very fast for the larger-sized nano-clusters. The biocompatible magnetic nano-clusters are stable in aqueous and physiological media as well as in blood plasma and blood serum. The small sized magnetic nano-clusters containing iron oxide-boron show magnetic self-arrangement in dispersions. The features of the magnetic nano-clusters offer a large application spectrum from technical and medical areas and are primarily suitable to enter the composition of pharmaceutical products for the cancer therapies Magnetic Drug Targeting and Boron Neutron Capture Therapy.

## Description

Magnetic nano-clusters containing iron oxide respectively iron oxide-boron can be prepared by wet chemistry having a spherical-like shape and size distributions around 200 nm, 150 nm, 100 nm, 50 nm, 15 nm and 5 nm depending on the preparation conditions. For the large-sized distributions the size variation is about 50 %. For the 15 nm and 5 nm distributions the size is uniform. The boron content is tunable in the range 10⁻⁴ wt% (ppm) to 10⁻¹ wt%. The magnetic nano-clusters consist of close packed small grains (nanoparticles) of iron oxide respectively iron oxide-boron. The grains are contained in an amorphous iron-based matrix. The close packing favors a large magnetic moment per nano-cluster. The large-sized nano-clusters collect extremely fast in moderate external magnetic fields. For a field maximum of 1-2 T the collection takes some seconds only. The saturation magnetization is typically between 35 and 64 emu/g and favors the large-sized magnetic nano-clusters to remain homogeneously dispersed in solvents. Boron increases the magnetic properties and the small-sized nano-clusters containing iron oxide-boron usually present in dispersions a magnetic self-arrangement. The magnetic nano-clusters containing iron oxide respectively iron oxide-boron are chemically non-toxic and become hydrophilic after modifying the matrix with organic molecules. The modified magnetic nano-clusters are biocompatible, being stable at least in water, physiological NaCl 0.9 % solution, blood plasma and blood serum.

The fast response to external magnetic fields and the stability in aqueous and physiological media make the large-sized magnetic nano-clusters containing iron oxide respectively iron oxide-boron primarily appropriate to enter in the composition of pharmaceutical products for the Magnetic Drug Targeting respectively Boron Neutron Capture Therapy.

The Magnetic Drug Targeting abbreviated MDT is an experimental therapy that uses magnetic vectors like vesicles and nanoparticles to magnetically target drugs in localized tumours or diseases. The maximum of an external magnetic gradient is positioned at the tumour place. The external magnetic gradient field can be generated for example by a large electromagnet having one of the pole in a V form, by small commercial FeBNd magnets or by flexible electromagnets specially designed for the magnetic targeting. The magnetic vectors are injected under the form of a pharmaceutical aqueous colloid usually via a blood vessel that lies near the tumour. The tiny size of the vectors allows them to travel through the blood vessels and the interaction with the external magnetic gradient produces their accumulation precisely at the tumour site. The therapy aims to target drugs in the affected tissues alone while the healthy tissues are avoided. Several problems like aggregation of the vectors at the contact with the blood and small response to the magnetic field are encountered. The magnetic nano-clusters containing iron oxide respectively iron oxide-boron could function as stable magnetic vectors and with a high response to the external magnetic fields. The matrix with the close packed grains can be loaded with drugs of medical interest in order to deposit them at the target place.

The Boron Neutron Capture Therapy abbreviated BNCT is another experimental therapy that uses local neutron irradiation of the tumour tissue after its enrichment with boron containing molecules. Upon neutron irradiation, the boron atoms are generating short-path energetic α particles and decay into lithium atoms. The BNCT is based on the following reaction: ¹⁰B (n, α) →¹⁰Li + 2.31 MeV. The generated α particles locally lose their energy on a very small distance of around 20 µm length, which is comparable with the length of 1-2 cancerous cells. The local therapeutic effect of BNCT is given by the deposited energy that destroys the cancerous cells. In BNCT it is still a problem to delimitate between the healthy and cancerous tissues during the administration of boron containing molecules. Also the deposited boron is usually not sufficient for an efficient therapy. In the case of localized tumours, biocompatible magnetic nano-clusters containing iron oxide-boron are suitable candidates to solve the BNCT problems. They can be magnetically accumulated like for the Magnetic Drug Targeting to selectively enrich the tumour with boron. The boron amount provided by the accumulated nano-clusters could significantly improve the efficiency of the BNCT therapy.

Other uses for the magnetic nano-clusters containing iron oxide respectively iron oxide-boron can be in contrast imaging, in hyperthermia, in diagnostics, in biological separations as well as in technical engineering i.e. magnetic coating, magnetic memories and magnetic elements.

The biocompatible magnetic nano-clusters containing iron oxide respectively iron oxide -boron are prepared by wet chemistry in polyol using a specially adapted synthesis method. As polyol it is used the non-toxic polyol propylene glycol. Other polyols e.g. ethylene glycol, polyethylene glycol, buthylene glycol are excluded due to their toxicity. The preparation continues with a subsequent treatment of the nano-cluster matrix to make it hydrophilic and biocompatible.

The preparation starts with a 300 ml volume of liquid propylene glycol (e.g. Carl Roth, Germany) that is poured in a round glass flask at room temperature. The flask has to be suitable for the connection to a nitrogen gas flow. A very small volume of 200 µl distilled filtered water is added in the propylene glycol and well mixed with it. The mixture propylene glycol - water is connected for some minutes to the nitrogen gas stream in order to partly remove the oxygen gas that is usually dissolved in the propylene glycol. The oxygen gas, controlled by an oxygen gas electrode, is typically removed between 0% and 80% depending on the aimed size of the nano-clusters. After each opening - e.g. for the addition of the chemicals or for the control measurements, the flask with the propylene glycol mixture is shortly reconnected to the nitrogen gas flow. In the flask with the propylene glycol mixture there are added upon stirring around 0.3ml solution of a 10 M NaOH for the iron oxide -boron nano-clusters and around 0.9 ml solution of a 10 M NaOH solution for the iron oxide nano-clusters. Successively there are added 3.8 ml of a 2 M FeCl₂ solution. The solutions and the distilled water used for their preparation are filtered down to some 200 nm. The total iron concentration in propylene glycol has to remain around 25 mM and the total water volume in propylene glycol has to remain reduced. The pH value after the addition of the two solutions can be carefully further adjusted by a NaOH 10 M solutions. The typical pH values for the preparation of the nano-clusters containing iron oxide are in the range between 7.0 and 10.0. The typical pH values for the preparation of the nano-clusters containing iron oxide-boron are in the range between 6.0 and 8.5. The preparation continues for the iron-oxide nano-clusters with the carefully addition of sodium borohydride NaBH₄ (e.g. Sigma Aldrich, Germany) in an amount between some hundreds of mg and 1 g . High boron amounts in the nano-clusters (10⁻¹ wt %) have been obtained for the preparations when up to 4 ml distilled water was added towards the end of the NaBH₄ reaction. It is suspected that either the addition of around 2-5 ml distilled water right after adding the NaBH₄ powder or using the NaBH₄ under the form of a fresh aqueous solution with a 2-5 ml volume would significantly increase the boron amount in the nano-clusters while favoring the large-sized ones. After the complete reaction of the NaBH₄ the flask with the propylene glycol mixture is connected to a permanent nitrogen gas flow. For the nano-clusters containing iron oxide the flask is connected to the permanent nitrogen gas flow right after adjusting the pH. Typically, the nitrogen gas flow remains adjusted at about 100-130 nitrogen bubbles per minute. The exit of the nitrogen gas tubing is optionally placed in a reducing mixture formed by about 200 ml propylene glycol and about 2 g Na₂S₂O₄. The flask connected to the nitrogen gas is introduced in a silicon oil bath contained in a Teflon pot and is heated at 110° C for typically 48 hours. During heating the propylene glycol mixture is continuously stirred i.e. by a magnetic stirrer. A supplementary control of the nitrogen gas flow is made after the temperature increase of the propylene glycol mixture. The heating is stopped when a deposit of black, opaque magnetic material starts to attach on the flask walls at the surface of the propylene glycol. In several cases the formed magnetic material gets collected on the magnetic stirring bar too. The magnetic material represents the formed nano-clusters. The obtained product is let to cool down and it is kept away from any contamination with water.

The size of the prepared nano-clusters increases with the decreasing velocity of their formation in propylene glycol. The reaction is significantly slowed down by reduced oxygen dissolved at the beginning and by a reduced oxygen flux during the heating step. The size of the nanoclusters is also affected by the water molecules in the propylene glycol mixture. Water forms nucleation and growth centers for the iron oxide respectively iron oxide-boron grains. A large water volume will determine numerous nucleation centers and accordingly numerous small nano-clusters. A very high nitrogen gas flow can stop the reaction responsible for the formation of the nano-clusters. The boron content in the nano-clusters slowly increases with the increase of the pH and amount of the NaBH₄ and more significantly with the water present at the moment of the NaBH₄ decomposition reaction. For the nano-clusters containing iron oxide there were occasionally added some 200 µl of 20 mg/ml solution of Na₂S₂O₄ to slow down the formation reaction of the nano-clusters.

The formed hydrophobic nano-clusters are separated from the propylene glycol mixture by centrifugation means. The product is diluted in proportion of 50 % vol. with ethanol and is centrifuged for 20 minutes with moderate centrifugal force (smaller than 3500 g). The obtained magnetic black pellet representing the nano-clusters is re-dispersed in ethanol and it is newly centrifuged. Between the centrifugation runs, the nano-clusters are dispersed in ethanol and stirred for 20 minutes. For long time storage the nano-clusters are dried by the freeze-dry technique. A phosphodextran aqueous solution (for the preparation see **Appendix 1)** with a concentration of about 2mg/ml it is prepared with distilled water and finally it is filtered. The solution is salt-free, it usually has an electrical conductivity smaller than 500 µS its pH is between 3.0 and 4.0 and has a volume of about 200 ml. An aliquot of about 5 ml of diluted ethanol suspension of nano-clusters is carefully dropped into the phosphodextran aqueous solution in a region close to the aqueous surface and under continuous stirring. The phosphodextran molecules spontaneously attach on the matrix of the nano-clusters. The attachment is irreversible. The modified nano-clusters are recovered from the phosphodextran aqueous mixture by centrifugation. The modified nano-clusters are hydrophilic and can be dispersed in distilled water, aqueous and physiological media, blood plasma and blood serum. Alternatively, the modified nano-clusters can be freeze-dried. The synthesized nano-clusters offer the possibility to be modified with other organic and inorganic molecules. A mixture fluorescein-isothiocyanate-phosphodextran fluorescently labels the nano-clusters. Besides phosphodextran, at least carboxydextran, aminodextran and albumin are shown to attach on the nano-cluster matrix too and to make the nano-clusters hydrophilic. Upon modification with organic molecules the hydrodynamic size of the hydrophilic nano-clusters in dispersions usually increases up to 50 %.

The phosphodextran modified nano-clusters can be loaded with the anticancer drug mitoxantrone. The nano-clusters are dispersed in distilled water, dropped in a highly diluted mitoxantrone solution (about 50 µg/ml) having a pH between 7.0 and 12.0 and finally separated by a short centrifugation. The mitoxantrone irreversibly attaches on the phosphodextran treated nano-clusters. It is suspected that the hydrophobic nano-clusters can be directly loaded with mitoxantrone. The phosphodextran modified nano-clusters loaded with mitoxantrone can be dispersed in aqueous and physiological media, blood plasma and blood serum.

Filtration, ultra-filtration, magnetic separation, size exclusion chromatography and buffer exchange may be used to purify the modified nano-clusters and the nano-clusters loaded with mitoxantrone. Increasing the solvent viscosity might avoid the slow sedimentation of the large-sized nano-clusters in dispersions. To avoid oxidation effects on a long period (noticeable after 1 month) it is indicated to keep the nano-clusters in dried form in vacuum desiccators. In external magnetic fields of 1-2 T the magnetic nano-clusters form in dispersions reversible chain-like formations with a length of 10-20 µm. After removing the magnetic field, the nano-cluster chains are easily decomposed by e.g. thermal energy or stirring the dispersion.

### Example1:

300 ml propylene glycol
200 µl distilled, filtered water
∼80-90 % oxygen gas removal by nitrogen gas flow
0.3 ml of 10 M NaOH solution
3.8 ml of 2 M FeCl₂ solution
measured pH ∼ 6.0
shortly N₂ gas flow
1 g NaBH₄ powder
4 ml distilled water added towards the end of the NaBH₄ reaction in the propylene glycol mixture heating at 110 ° C for 18 hours

The preparation yielded 5 nm iron oxide - boron magnetic nano-clusters with a high boron content of 0.5 %.

### Example 2:

300 ml propylene glycol
200 µl distilled filtered water
∼80-90 % oxygen gas removal by nitrogen gas flow
0.5 ml of 10 M NaOH solution
3.8 ml of 2 M FeCl₂ solution
pH adjusted to value 7.0
shortly N₂ gas flow
1 g NaBH₄ powder
heating at 110 ° C for 48 hours

The preparation yielded 170 nm iron oxide - boron magnetic nano-clusters with a boron content of ∼ 100 ppm.

### Example3:

300 ml propylene glycol
200 µl distilled filtered water
∼60-80 % oxygen gas removal by nitrogen gas flow
0.3 ml of 10 M NaOH solution
3.8 ml of 2 M FeCl₂ solution
pH adjusted to value 6.5
2 minutes N₂ gas flow
1g NaBH₄ powder
heating at 110 ° C for 20 hours

The preparation yielded 80 nm iron oxide - boron magnetic nano-clusters with a boron content of ∼ 200 ppm.

### Example 4:

300 ml propylene glycol
200 µl distilled filtered water
no oxygen gas removal
0.65 ml of 15 M NaOH solution
3.8 ml of 2 M FeCl₂ solution
measured pH ~ 7.0
0.5 g NaBH₄ powder
0.35 ml of 15 M NaOH solution
heating at 110 ° C for 10 hours

The preparation yielded black magnetic nano-clusters with 5 nm, with a boron content of 143 ppm. The sample shows typical magnetic self arrangement of the nano-clusters in ethanol.

### Example 5:

300 ml propylene glycol
200 µl distilled filtered water
∼60 % oxygen gas removal by nitrogen gas flow
0.9 ml of 15 M NaOH solution
3.8 ml of 2 M FeCl₂ solution
measured pH > 8
2 minutes N₂ gas flow
0.12 g NaBH₄ powder
heating at 110 ° C for 23 hours

The preparation yielded black magnetic nano-clusters with 5 nm, with the boron content of 460 ppm.

Example 6:
300 ml propylene glycol
200 µl distilled filtered water
∼80 % oxygen gas removal by nitrogen gas flow
1 ml of 15 M NaOH solution
3.8 ml of 2 M FeCl₂ solution
measured pH > 8
2 minutes N₂ gas flow
heating at 110 ° C for 17 hours

The preparation yielded black magnetic nano-clusters containing iron oxide, with a size around 100 nm.

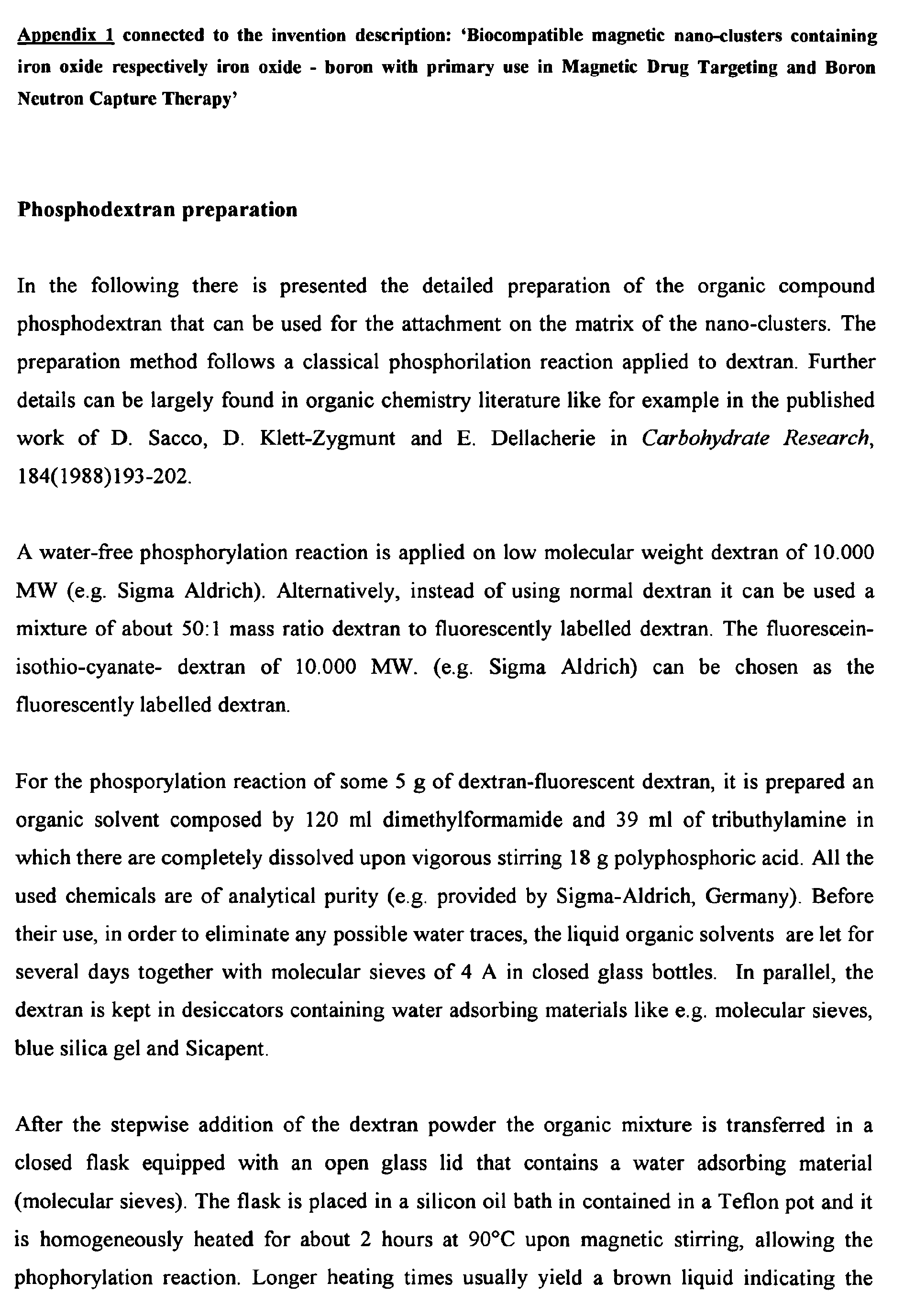

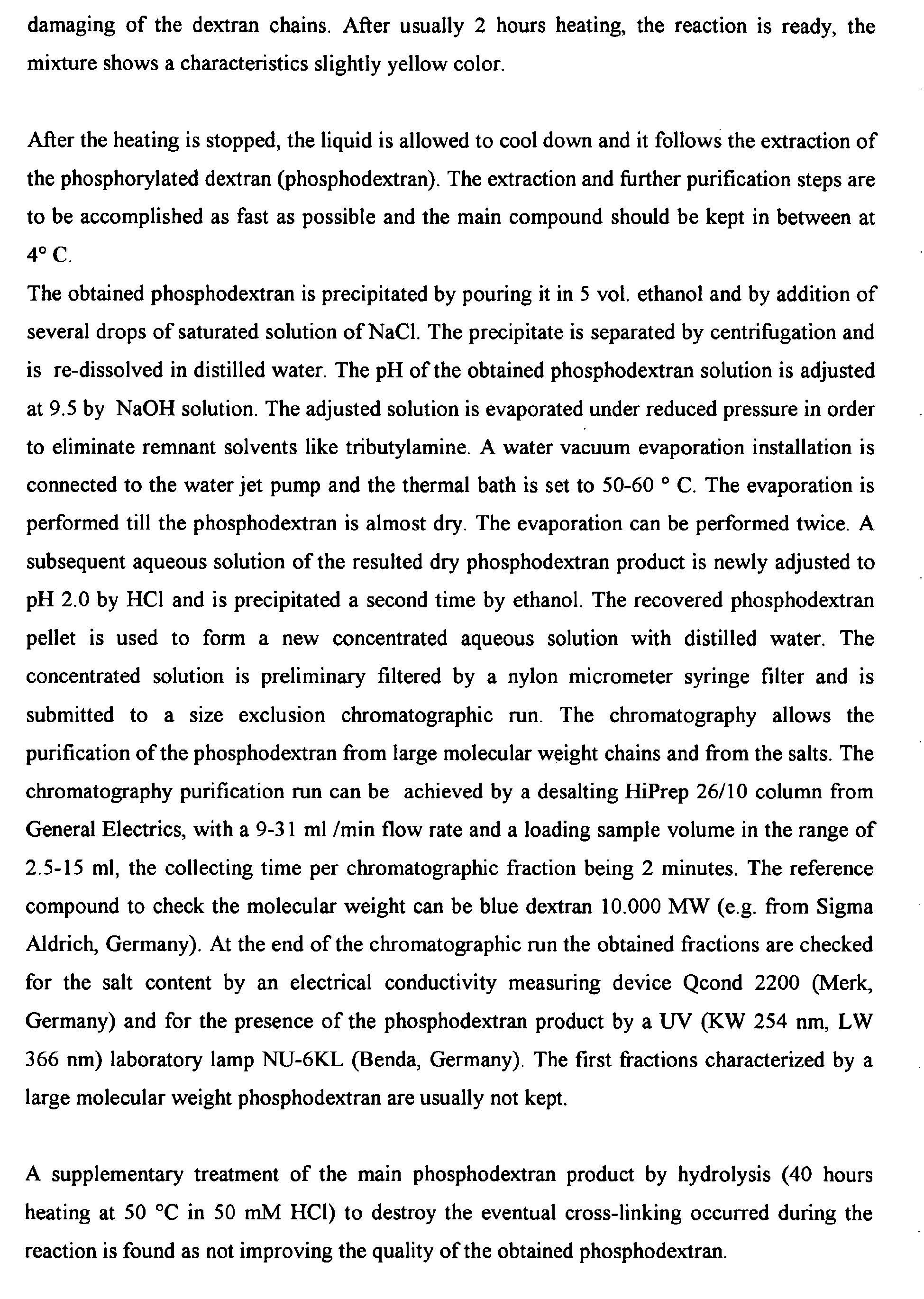

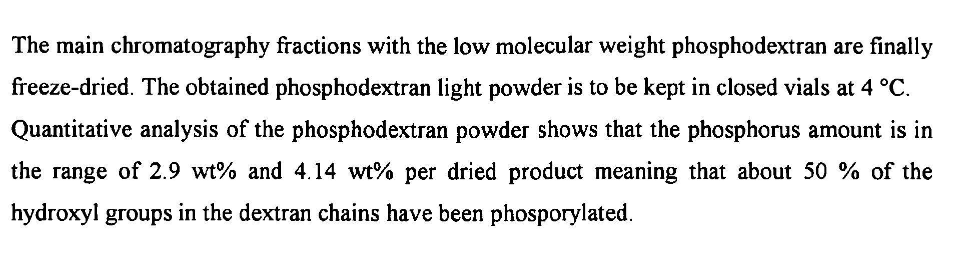

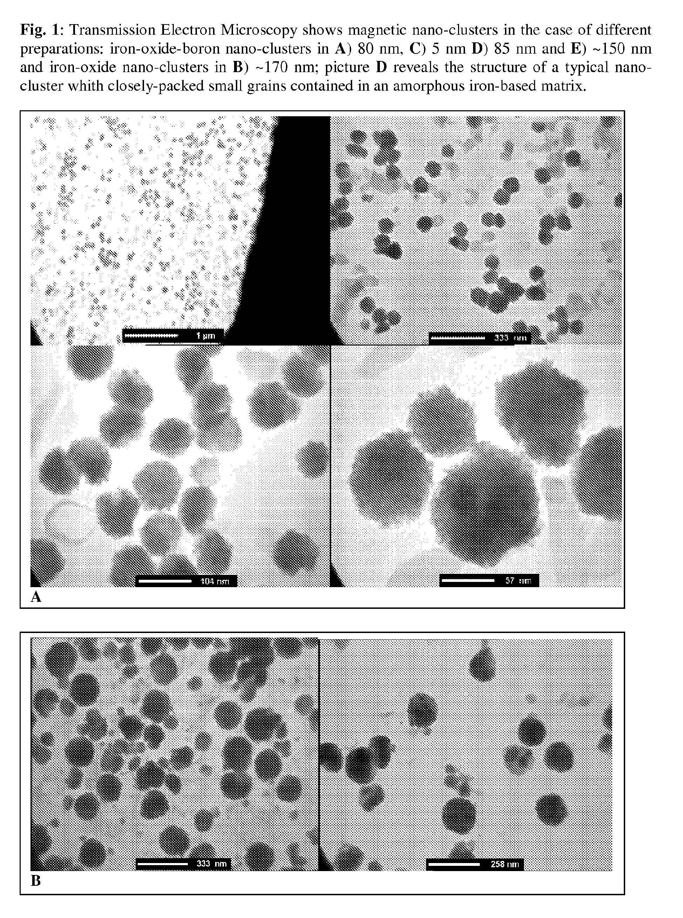

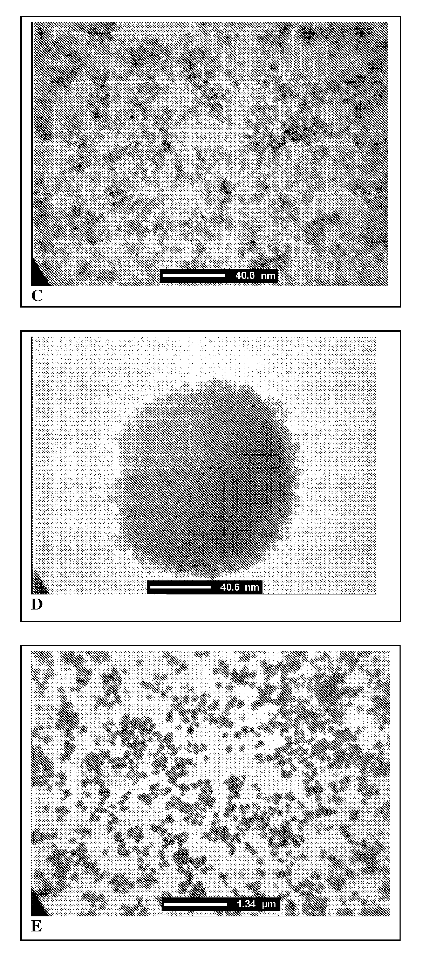

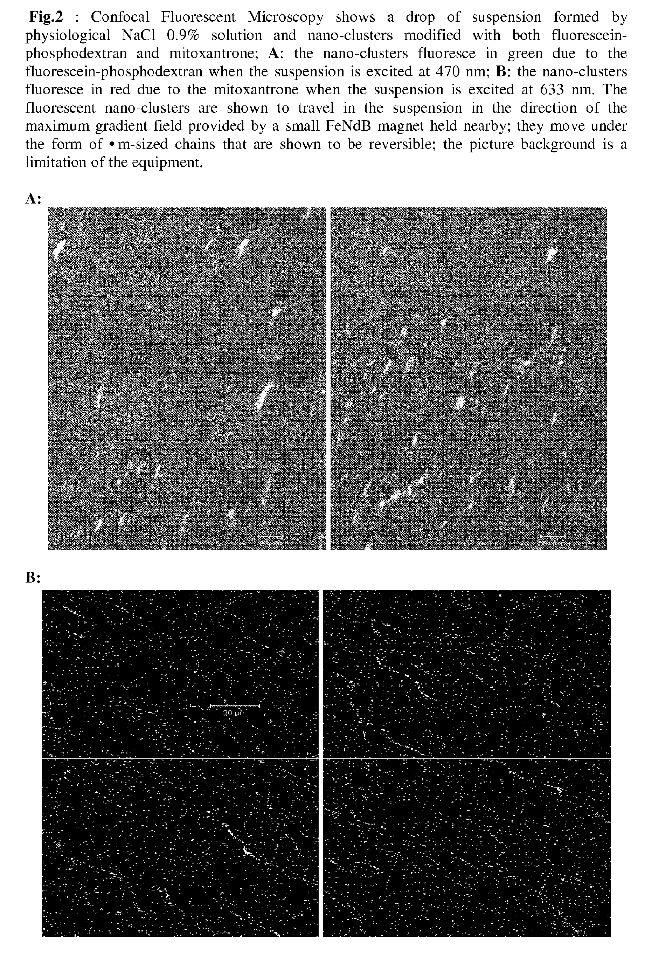

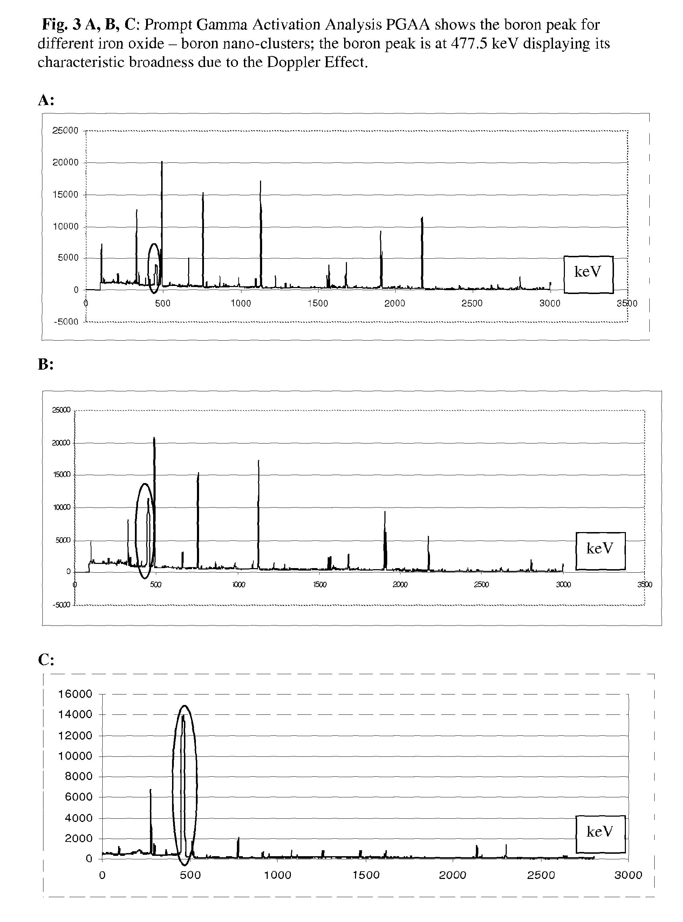

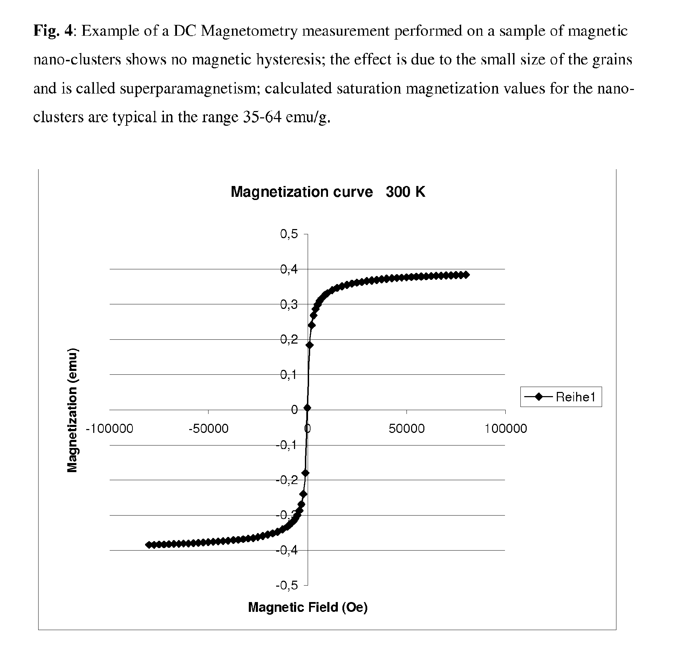

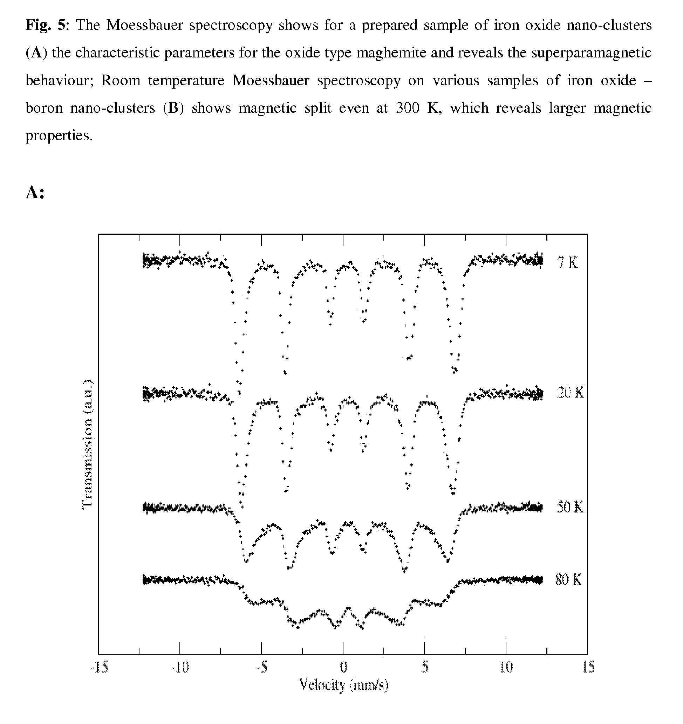

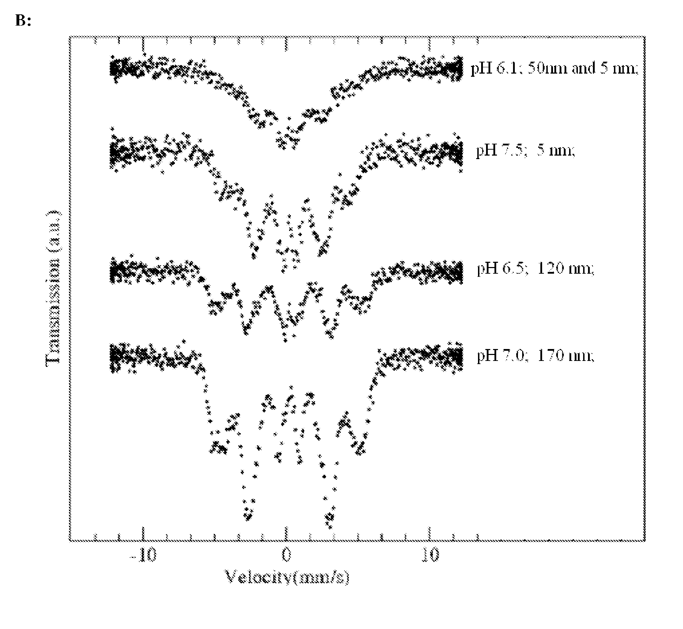

## Claims

1. Hydrophobic non-toxic magnetic nano-clusters containing iron oxide with tunable size distributions around 200 nm, 150 nm, 100 nm, 50 nm, 15 nm and 5 nm having a large magnetic moment per nano-cluster and obtained by wet chemistry in propylene glycol under nitrogen gas flow and controlled pH.

2. Hydrophobic non-toxic magnetic nano-clusters containing iron oxide - boron with tunable size distributions around 200 nm, 150 nm, 100 nm, 50 nm, 15 nm and 5 nm having a large magnetic moment per nano-cluster and obtained by wet chemistry in propylene glycol under nitrogen gas flow and controlled pH and using sodium borohydride NaBH₄.

3. The nano-clusters set forth in claim 1 and claim 2 are formed by close packed small grains of iron oxide respectively iron-oxide boron contained in an amorphous iron-based hydrophobic matrix.

4. Hydrophilic and biocompatible magnetic nano-clusters containing iron oxide and iron oxide - boron obtained by attaching phosphodextran, carboxydextran, aminodextran and albumin on the nano-clusters set forth in claim 1 and claim 2.

5. Fluorescently labelled hydrophilic magnetic nano-clusters containing iron oxide and iron oxide-boron obtained by attaching fluorescein-isothiocyanate phosphodextran on the nano-clusters set forth in claim 1 and claim 2.

6. Hydrophilic mitoxantrone loaded magnetic nano-clusters containing iron oxide and iron oxide - boron obtained by supplementary attaching mitoxantrone on the hydrophilic magnetic nano-clusters set forth in claim 3.

7. Hydrophilic mitoxantrone loaded and fluorescently labelled magnetic nano-clusters containing iron oxide and iron oxide - boron obtained by supplementary attaching mitoxantrone on the fluorescent labelled nano-clusters set forth in claim 4.

8. Mitoxantrone loaded magnetic nano-clusters by attaching mitoxantrone on the nano-clusters set forth in claim 1 and claim 2.

9. Simple aqueous dispersions of mitoxantrone loaded magnetic nano-clusters set forth in claim 7 where the solvents are aqueous and physiological solutions, dextran solutions and blood serum and blood plasma.

10. Simple aqueous dispersions of hydrophilic and biocompatible magnetic nano-clusters set forth in claim 3 and claim 4 where the solvents are aqueous and physiological solutions, dextran solutions and blood serum and blood plasma.

11. Simple aqueous dispersions of mitoxantrone loaded magnetic nano-clusters set forth in claim 5 and claim 6 where the solvents are aqueous and physiological solutions, dextran solutions and blood serum and blood plasma.

12. Ethanol dispersions of hydrophobic magnetic nano-clusters set forth in claim 1 and claim 2.

13. Powders and sintered powders of hydrophobic magnetic nano-clusters set forth in claim 1 and claim 2.

14. Powders and sintered powders of hydrophilic magnetic nano-clusters set forth in claims 3 - 4 and claims 5 - 7.

15. Polyol dispersions of hydrophobic magnetic nano-clusters set forth in claim 1 and claim 2.
